# EUROPÄISCHE PATENTSCHRIFT

(11) **EP 2 359 870 B1**
(45) Veröffentlichungstag und Bekanntmachung des Hinweises auf die Patenterteilung: **20.03.2013**
(21) Anmeldenummer: 10014127.4
(22) Anmeldetag: 29.10.2010
(51) Int. Cl.: A61L 2/07

(54) **Desinfektionsverfahren für Klimaschränke**
Sterilisation method for air-conditioning chambers
Procédé de désinfection pour armoires climatiques

(30) Priorität: 26.01.2010 DE 102010005748
(43) Veröffentlichungstag der Anmeldung: 24.08.2011
(73) Patentinhaber: Thermo Electron LED GmbH, 63505 Langenselbold (DE)
(72) Erfinder: Betz, Stefan, 63526 Erlensee (DE); Loscher, Helmut, 61130 Nidderau (DE); Heeg, Hubert, 63739 Aschaffenburg (DE)
(74) Vertreter: Lang, Friedrich

(56) Entgegenhaltungen:
- EP-A1- 1 484 069
- EP-A1- 1 650 291
- EP-A2- 0 923 946
- US-A- 4 336 329

## Beschreibung

Die Erfindung betrifft ein Desinfektionsverfahren für Klimaschränke.

Bei der Desinfektion von modernen Klimaschränken, Inkubatoren und Brutschränken mit karussellartigen Objektträgereinbauten und sensorisch sowie per Computer und Roboter gesteuerten Transportsystemen besteht die Notwendigkeit, die Innenräume möglichst mit den darin angeordneten Einbauten in regelmäßigen Abständen zu reinigen, ohne die Einbauten einer zu großen Belastung zu unterwerfen.

Insbesondere moderne Klimaschränke sind mit komplexen Robotics-Systemen ausgestattet, die zur Ein- und Auslagerung der Behältnisse, beispielsweise von Mikrotiterplatten und Probenträgern, eine sehr große und zerklüftete Oberfläche aufweisen. Ein Beispiel für einen solchen modernen Klimaschrank ist in der WO 98/05753 A1 beschrieben. Aufgrund der ausgezeichneten Wachstumsbedingungen für Keime und Mikroorganismen während des Betriebs solcher Geräte können diese Oberflächen der Einbauten auch für unerwünschte Mikroorganismen eine gute Basis bilden.

Die Oberflächen sind aufgrund ihrer Komplexität jedoch für eine händische Säuberung und Sterilisation nahezu nicht zugänglich, so dass auf ein automatisiertes Verfahren zurückgegriffen werden muss. Herkömmlich eingesetzte Desinfektionsgase wie Ethylenoxid oder Formaldehyd stellen wegen der damit verbundenen Gesundheitsgefahr stets ein unerwünschtes Risiko dar, und eine Begasung mit Wasserstoffperoxid ist zudem apparativ sehr aufwendig.

Bisher bekannte Verfahren zur Desinfektion von solchen Systemen, wie sie beispielsweise in der US 5,196,165 A, EP 1 650 291 A1 oder in der DE 44 06 632 C1 beschrieben sind, sehen jeweils eine Heißdampf-Sterilisation unter hohen Temperaturen von mehr als 120 °C vor. Hinsichtlich der modernen Einbauten mit hitzeempfindlichen Motoren, Robotern und Sensoren haben sich diese Verfahren jedoch als nicht ausreichend geeignet herausgestellt, um die Einbauten dauerhaft betriebsfähig zu halten. EP 1 484 069 A1 beschreibt die Autoklavendesinfektion von Endoskopen, US 4,336,329 A ein Trockensterilisationsverfahren eines Klimaschrankes bei ca. 180 °C.

Daneben ist aus der europäischen Patentschrift EP 0 923 946 B1 ein Verfahren zur Desinfektion von Begasungsbrutschränken bekannt, das bei einer verhältnismäßig niedrigen Temperatur von 90 °C und einer relativen Feuchte von mindestens 80 % arbeitet. Der relativ langen Desinfektionsphase von mindestens 9 Stunden schließt sich hier zwingend eine Kondensationsphase an, in der durch Kühlung der Bodenwand eine Kondensation des eingebrachten Wassers erzielt wird. Es wird weiterhin berichtet, dass zur Vermeidung von Wasser-Kondensation an der transparenten Innentür eine Heizung an dieser Tür vorgesehen ist. Eine Kondensation an den empfindlichen Einbauten kann dagegen nicht verhindert werden, da diese nicht wie eine Tür oder wie eine glatte Innenwand beheizt werden können. Somit kondensiert bei dem beschriebenen Verfahren in jedem Fall Wasser an den empfindlichen Einbauten und kann damit leicht zu einer unerwünschten Verkürzung der Lebenszeit der Einbauten führen.

Nachteilig bei allen bisher bekannten Verfahren zur Desinfektion beziehungsweise Sterilisation von gattungsgemäßen Brutschränken ist mithin, dass sie keine Möglichkeit bieten, die hitze- und feuchteempfindlichen Einbauten moderner Geräte ausreichend zu schützen, um einer Verkürzung ihrer Lebensdauer entgegen zu wirken.

Aufgabe der vorliegenden Erfindung ist es daher, ein Verfahren zur Desinfektion von Klimaschränken bereitzustellen, das auch die besonderen Anforderungen hinsichtlich hitze- und feuchteempfindlicher Einbauten wie Roboter-Transportsysteme, Motoren und Sensoren optimal erfüllt. Zudem soll das Verfahren gleichzeitig in der Lage sein, die Verfahrensdauer der Desinfektion unter relativ niedrigen Temperaturen zu minimieren.

Die Aufgabe wird erfindungsgemäß gelöst durch das Verfahren nach Anspruch 1. Bevorzugte Verfahrensvarianten sind in den Unteransprüchen beschrieben. Die Erfindung betrifft zudem die Verwendung des Verfahrens nach Anspruch 8.

Das Verfahren zur Desinfektion eines Innenraumes eines Klimaschranks umfasst die Schritte
i) Erzeugung einer desinfizierenden, heißen Atmosphäre im zu desinfizierenden Raum durch Wärmezufuhr und Verdampfung von Wasser in einer Aufheizphase,
ii) nach Erreichen einer Temperatur von 95 ± 5 °C und einer relativen Feuchte von mindestens 80 % bei einem dem Druck der Umgebungsatmosphäre entsprechenden Druck Halten der vorgenannten Verfahrensbedingurigen in einer Desinfektionsphase für einen zur Abtötung von im zu desinfizierenden Raum befindlichen Keimen ausreichenden Zeitraum, und
iii) anschließend Austreiben der relativen Feuchte aus dem zu desinfizierenden Raum und Abkühlen in einer Abkühlphase, wobei der zur Desinfektion erzeugte Wasserdampf durch Einblasen steriler Luft in der Abkühlphase aus dem zu desinfizierenden Raum entfernt wird.

Erfindungsgemäß kann aufgrund der hochfeuchten Atmosphäre eine gegenüber trockener Atmosphäre erhöhte Wärmesensibilisierung der vorhandenen Keime erzielt werden, so dass sich diese in einer zweckmäßig mehrstündigen Desinfektionsphase vorteilhafterweise praktisch alle thermisch abtöten lassen. Sinnvoller Weise kann die Desinfektionsphase in die Nacht oder auf ein Wochenende gelegt werden, damit nicht unnötig Arbeitszeit verloren geht. Das erfindungsgemäße Verfahren vermeidet zudem vorteilhafterweise gesundheitsschädliche oder apparativ aufwendige Desinfektionsgase wie Ethylenoxid, Formaldehyd oder Wasserstoffperoxid.

Außerdem kann durch das erfindungsgemäße Einblasen von steriler Luft zum Austreiben des Wasserdampfs in der Abkühlphase eine unerwünschte Kondensation von Wasser an den Feuchte empfindlichen Einbauten und am Boden vermieden werden. Dadurch gelingt es mit dem erfindungsgemäßen Verfahren erstmals, die Lebenszeit der Einbauten nicht aufgrund von Kondensation und damit verbundener möglicher Korrosion zu verringern. Um den gewünschten Erfolg zu erreichen, wird die Temperatur im zu desinfizierenden Raum zweckmäßig so langsam abgesenkt, dass zunächst die Feuchtigkeit im zu desinfizierenden Raum soweit durch Einblasen steriler Luft reduziert ist, das sie beim Absinken der Temperatur unter den Kondensationspunkt nicht mehr für eine nennenswerte Kondensation ausreicht.

Weiterhin vorteilhaft kann die Verfahrensdauer aufgrund des Austreibens des Wasserdampfs mittels steriler Luft minimiert werden gegenüber den bisherigen Verfahren mit langen Kondensationsphasen und nachfolgenden Nachheizphasen, in denen Restkondensat entfernt werden muss.

Daneben lässt sich das erfindungsgemäße Verfahren sehr gut automatisieren, so dass ein händisches Eingreifen von Bedienpersonen während des Verfahrens nicht mehr erforderlich ist. Dazu werden bevorzugt bestimmte Messwerte im zu desinfizierenden Innenraum des Klimaschrankes bestimmt, insbesondere die Feuchtigkeit und Temperatur. Gegebenenfalls kann auch der Druck innerhalb des Innenraums ermittelt werden. Für diese Messparameter sind zweckmäßig in einer Speichereinheit Sollwerte für die verschiedenen Phasen des Desinfektionsverfahrens hinterlegt. Die ermittelten Istwerte werden in einer Auswertungseinheit mit dem entsprechenden Sollwert verglichen. Werden Abweichungen von den Sollwerten festgestellt, erfolgt zweckmäßig mittels einer Regeleinheit eine Nachregelung, um den Wert in den gewünschten Bereich zu bringen.

Konkret werden beispielsweise in Schritt i) Temperatur und Feuchtigkeit im Innenraum gemessen, und die Temperatur wird solange erhöht, bis sich die in Schritt ii) genannten Temperatur- und Feuchtigkeitswerte eingestellt haben. Bevorzugt wird eine Temperatur von genau 90 °C eingestellt. Daraufhin wird beispielsweise ein Timer gestartet, der die Zeit misst, die für Schritt ii) vorgesehen ist (beispielsweise 5 bis 12 Stunden). Zudem werden Temperatur und Feuchtigkeit weiter gemessen, und es wird weiter nachgeregelt, damit die vorgegebenen Bedingungen über die für Schritt ii) vorgesehene Zeit eingehalten werden. Dabei können natürlich gewisse Korridore vorgesehen werden, innerhalb derer eine Abweichung von den vorgegebenen Werten zulässig ist. Nach Ablauf der vorgegebenen Zeit wird Schritt ii) beendet und Schritt iii) gestartet. Auch während der Abkühlphase wird zweckmäßig zumindest die Luftfeuchtigkeit und gegebenenfalls die Temperatur im Innenraum weiter gemessen. Sterile Luft wird solange eingeblasen, bis die Feuchtigkeit einen vorgegebenen Wert erreicht hat. Danach wird das Desinfektionsverfahren beendet. Der Normalbetrieb des Klimaschrankes kann wieder aufgenommen werden. Die für die Messung erforderlichen Messgeräte wie ein Thermometer sowie ein Hygrometer sind üblicherweise in herkömmlichen Klimaschränken, insbesondere in Inkubatoren, zur Bestimmung dieser Parameter im Innenraum ohnehin vorhanden.

Die sterile Luft zum Austreiben des Wasserdampfs in der Abkühlphase kann in einer vorteilhaften Ausgestaltung des erfindungsgemäßen Verfahrens über Sterilfilter aus der Umgebungsluft erzeugt werden. Damit kann auf einfache und kostengünstige Weise eine ausreichende Versorgung mit steriler Luft sichergestellt werden.

Vor dem Desinfektionsvorgang wird das zu desinfizierende Gerät zweckmäßig abgeschaltet, eventuell vorhandenes Kulturgut oder Proben werden aus dem zu desinfizierenden Raum entfernt sowie der Wasservorrat zur kontrollierten Befeuchtung der Atmosphäre während der normalen Gebrauchsphase entleert. Zudem können wenn nötig manuelle Reinigungsarbeiten vorgenommen werden.

Das erfindungsgemäße Verfahren kann bevorzugt zur Desinfektion von Klimaschränken, Inkubatoren und Brutschränken, umfassend elektronische Einbauten und Sensoren, verwendet werden. Gerade bei diesen modernen Laborgeräten mit hitze- und feuchteempfindlichen Einbauten kann eine Desinfektion des gesamten Nutzraums mit allen Einbauten schonend vorgenommen werden. Zudem kann die Verfahrensdauer durch Einsparung von bisher notwendigen Kondensations- und Nachheizphasen erheblich verkürzt werden.

Das erfindungsgemäße Verfahren eignet sich speziell für einen Klimaschrank, der eine karussellartige Objektlagervorrichtung und mindestens eine Transporteinrichtung, die durch Roboter und Sensoren gesteuert und betrieben werden, umfasst, wobei der Klimaschrank eine Vorrichtung zur Erzeugung und Durchleitung von steriler Luft aufweist, um das erfindungsgemäße Verfahren auszuführen. Bevorzugt kann die Vorrichtung zur Erzeugung von steriler Luft mindestens einen Sterilfilter zur Sterilisation von angesaugter Umgebungsluft aufweisen. Auf diese Weise kann die zum Ausblasen des zur Desinfektion verwendeten Wasserdampfs benötigte sterile Luft einfach und kostengünstig aus der Umgebungsluft erzeugt werden.

In einer weiteren bevorzugten Ausgestaltung des Klimaschranks können Druckausgleichsmittel vorgesehen sein, die den Druck im Inneren des zu desinfizierenden Raums im Wesentlichen an den Druck der umgebenden Atmosphäre angleichen. Damit kann eine schonende Atmosphäre für die Einbauten sichergestellt werden, so dass Materialbelastungen durch unterschiedliche Drücke im Inneren gegenüber der äußeren Atmosphäre vermieden werden.

Die Erfindung wird im Folgenden anhand der Zeichnung näher erläutert, ohne die Erfindung darauf zu beschränken. Gleiche Bezugszeichen bezeichnen gleiche Teile. Die Abbildungen zeigen schematisch
- Fig. 1: eine perspektivische Ansicht eines erfindungsgemäßen Klimaschrankes und
- Fig. 2: ein Flussdiagramm zur Veranschaulichung des Ablaufs eines erfindungsgemäßen Desinfektionsverfahrens.

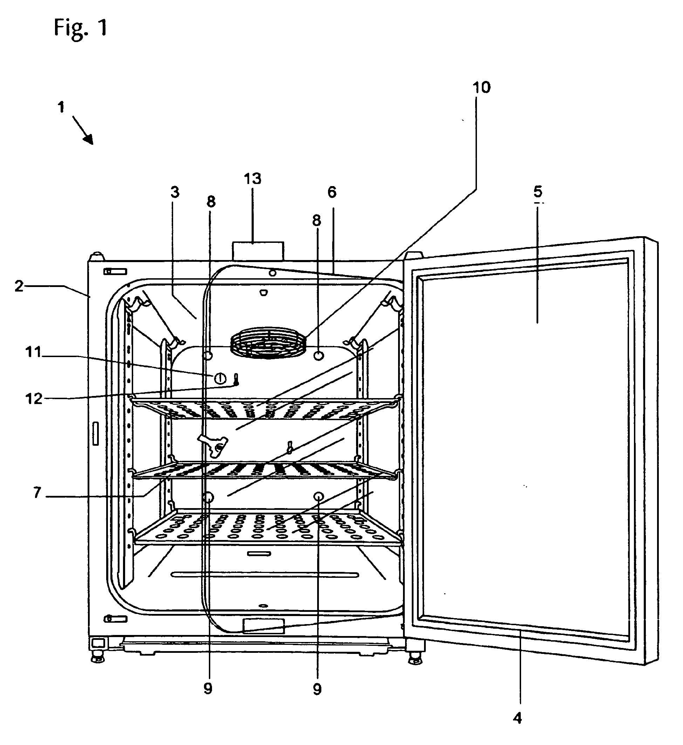

Fig. 1 zeigt eine Draufsicht auf einen Klimaschrank 1. Er weist ein schrankartiges Gehäuse 2 auf, das einen Innenraum 3 umgibt. In Richtung auf den Betrachter hin ist der Innenraum mit einer Tür 4 verschließbar. Zwischen Außentür 4 und Innenraum 3 ist eine weitere Tür 6 vorhanden, die in einer Vertiefung 5 einliegt, wenn beide Türen verschlossen sind. Die Innentür 6 besteht aus Glas und ermöglicht es, in den Innenraum 3 hineinzusehen, wenn die Tür 4 geöffnet ist. Die Innentür 6 verhindert dabei, dass ein zu schneller unerwünschter Austausch der Atmosphäre des Innenraums mit der Umgebung stattfindet. Der Innenraum dient zur Aufbewahrung von (hier nicht dargestellten) Proben, die auf Lagerböden 7 gelagert werden können. Um die Proben bei optimalen Bedingungen lagern zu können oder um im Falle eines Inkubators eine Inkubation der Proben durchführen zu können, werden im Innenraum 3 bestimmte Temperatur-, Feuchtigkeits- und gegebenenfalls Gaszusammensetzungsbedingungen (zum Beispiel eine Kohlendioxid-Atmosphäre) erzeugt und aufrechterhalten.

Die im Inneraum gelagerten mikrobiologischen Proben führen im Laufe der zeit zu einer Kontamination des Innenraums. Daher ist es in regelmäßigen Abständen notwendig, den Innenraum zu dekontaminieren. Erfindungsgemäß geschieht dies durch Desinfektion des Innenraumes mit sämtlichen seiner Einbauten (also zum Beispiel einschließlich der Lagerböden 7), aber natürlich ohne Proben, mittels Heißdampf. Hierzu wird in einem ersten Verfahrensabschnitt zunächst Wasser (zum Beispiel aus einem im Innenraum befindlichen Wasserbad) verdampft und erhitzt. Zur Vergleichmäßigung der Atmosphäre dient ein Ventilator 10 an der Innenraumdecke. Feuchtigkeit und Temperatur im Innenraum werden mit einem Hygrometer 11 bzw. einem Thermometer 12 gemessen. Die Messwerte werden an eine Speicher- und Auswertungseinheit weitergegeben, die im Gerät eingebaut oder extern angeordnet sein kann (hier stark schematisiert als 13 bezeichnet). Dort werden die Messwerte mit abgespeicherten Sollwerten verglichen. Stellt die Auswertungseinheit fest, dass die für die Aufheizphase vorgegebenen Sollwerte von Feuchtigkeit und Temperatur erreicht sind, gibt eine Regeleinheit (die zusammengefasst mit der Steuer- und Auswertungseinheit sein kann) das Signal zum Beginn des zweiten Verfahrensabschnitts.

Sind also im Innenraum 3 eine Temperatur von mindestens 95 ± 5 °C und eine Feuchtigkeit von mindestens 80 % erreicht, werden diese Bedingungen solange, in der Regel für mehrere Stunden, beibehalten, bis die Keime im Innenraum abgetötet sind. Dabei werden erneut Temperatur und relative Feuchtigkeit gemessen und die Messwerte mit den Sollwerten verglichen, um bei einer Abweichung eine Temperaturregelung durchzuführen und die Werte wieder in den gewünschten Bereich zu bringen. Mit Beginn der zweiten Phase wird die Zeit gemessen. Die Haltephase (Schritt ii)) wird nun für den vorgegebenen Zeitraum durchgeführt. Nach Ablauf der vorgegebenen Zeit wird Phase ii) beendet.

Nun folgt die Abkühlphase, in der das Klima im Innenraum wieder auf Normalbedingungen zurückgeführt wird. Zum Austreiben des heißen Wasserdampfes aus dem Innenraum wird durch Lufteinblasöffnungen 8 gefilterte Luft, bevorzugt sterile Luft, in den Innenraum eingeblasen und zunehmend entfeuchtete Luft durch die Auslassöffnungen 9 aus dem Innenraum abgezogen. Die Temperatur im Innenraum kühlt sich dabei allmählich ab. Durch Messung von Temperatur und Luftfeuchtigkeit im Verlauf der Abkühlphase kann festgestellt werden, wann die vorgegebenen Endwerte erreicht sind und das Desinfektionsverfahren beendet werden kann.

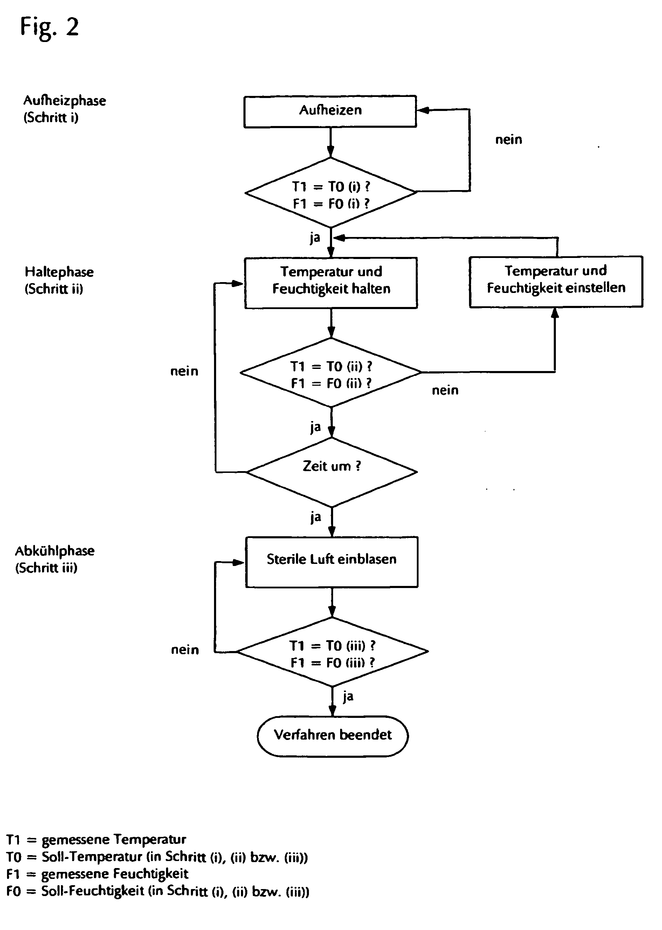

Die vorstehend beschriebenen verfahrensschritte sind in Form eines Blockdiagramms schematisch in Fig. 2 dargestellt.

## Patentansprüche

1. Verfahren zur Desinfektion von Klimaschränken (1) mit einer Aufheiz-, Desinfektions- und Abkühlphase, umfassend
i) Erzeugung einer desinfizierenden, heißen Atmosphäre im zu desinfizierenden Raum (3) durch Wärmezufuhr und Verdampfung von Wasser in der Aufheizphase,
**gekennzeichnet durch** die weiteren Schritte
ii) nach Erreichen einer Temperatur von 95 ± 5 °C und einer relativen Feuchte von mindestens 80 % bei einem dem Druck der Umgebungsatmosphäre entsprechenden Druck Halten der vorgenannten Verfahrensbedingungen in der Desinfektionsphase für einen zur Abtötung von im zu desinfizierenden Raum (3) befindlichen Keimen ausreichenden Zeitraum, und
iii) anschließend Austreiben der relativen Feuchte aus dem zu desinfizierenden Raum (3) und Abkühlen in der Abkühlphase,
wobei der zur Desinfektion erzeugte Wasserdampf **durch** Einblasen gefilterter Luft in der Abkühlphase aus dem zu desinfizierenden Raum (3) entfernt wird.

2. Verfahren nach Anspruch 1,
worin Schritt ii) für einen mehrstündigen Zeitraum ausgeführt wird.

3. Verfahren nach einem der vorhergehenden Ansprüche,
worin die gefilterte Luft zum Austreiben des Wasserdampfs in der Abkühlphase über Sterilfilter aus der Umgebungsluft erzeugt wird.

4. Verfahren nach einem der vorhergehenden Ansprüche,
worin während wenigstens einem der Schritte i), ii) und iii) wenigstens einer der folgenden Messwerte bestimmt und mit einem entsprechenden in einer Speichereinheit gespeicherten Sollwert verglichen wird:
• Temperatur im zu desinfizierenden Raum (3),
• Luftfeuchtigkeit im zu desinfizierenden Raum (3),
• Druck im zu desinfizierenden Raum (3).

5. Verfahren nach Anspruch 4,
worin bei Feststellung einer Abweichung des Messwerts vom Sollwert eine Regelung derart erfolgt, dass der Messwert dem Sollwert angeglichen wird.

6. Verfahren nach einem der vorhergehenden Ansprüche,
worin der Klimaschrank (1) eine Objektlagervorrichtung, insbesondere eine karussellartige Objektlagervorrichtung, und mindestens eine Transporteinrichtung, die durch Roboter und Sensoren gesteuert und betrieben werden, umfasst.

7. Verfahren nach einem der vorhergehenden Ansprüche,
worin der Druck im Inneren des zu desinfizierenden Raums (3) mittels eines Druckausgleichsmittels an den Druck der umgebenden Atmosphäre angeglichen wird.

8. Verwendung des Verfahrens nach einem der vorhergehenden Ansprüche zur Desinfektion von Klimaschränken umfassend elektronische Einbauten und Sensoren.

## Claims

1. Method for disinfection of climatic cabinets (1) involving a heating-up phase, a disinfection phase and a cooling phase, comprising:
i. generating a disinfecting hot atmosphere in the space to be disinfected (3) by supplying heat and evaporating water in the heating-up phase;
**characterized in that** it further comprises the following steps:
ii. when a temperature of 95 ± 5 °C and a relative humidity of at least 80% are reached at a pressure corresponding substantially to the pressure of the ambient atmosphere, maintaining these process conditions in the disinfection phase for a period of time which is sufficient for killing germs situated in the space to be disinfected (3); and
iii. subsequently expelling the relative humidity from the space to be disinfected (3) and cooling in a cooling phase,
wherein the water vapor generated for disinfection is removed from the space to be disinfected (3) by injecting filtered air in the cooling phase.

2. The method according to claim 1,
wherein step ii) is performed for a period of several hours.

3. The method according to any one of the preceding claims,
wherein the filtered air for the expulsion of the water vapor in the cooling phase is generated via sterile filters from ambient air.

4. The method according to any one of the preceding claims,
wherein at least one of the following measured values is determined and is compared with a corresponding set value stored in a memory unit during at least one of the steps i), ii) and iii):
- temperature in the space to be disinfected (3);
- air humidity in the space to be disinfected (3);
- pressure in the space to be disinfected (3).

5. The method according to claim 4,
wherein, in cases where a deviation of the measured value from the set value is determined, a control is carried out such that the measured value is assimilated to the set value.

6. The method according to any one of the preceding claims,
wherein the climatic cabinet (1) comprises an object storage device, in particular a carousel-type object storage device, and at least one transport apparatus that are controlled and operated by robots and sensors.

7. The method according to any one of the preceding claims,
wherein the pressure in the interior of the space to be disinfected (3) is assimilated to the pressure of the ambient atmosphere by use of pressure compensation means.

8. The use of the method according to any one of the preceding claims for disinfection of climatic cabinets comprising electronic installed elements and sensors.

## Revendications

1. Procédé pour la désinfection d'armoires climatiques (1) avec des phases de montée en température, de désinfection et de refroidissement, comprenant:
i. la création d'une atmosphère chaude désinfectante dans l'espace à désinfecter (3) par ap port de chaleur et évaporation d'eau pendant la phase de montée en température,
**caractérisé en ce qu'**il comporte en outre les étapes suivantes:
ii. quand une température de 95 ± 5°C et une humidité relative d'au moins 80 % sont atteintes à une pression correspondant à la pression de l'atmosphère ambiante, maintien des conditions de procédé susmentionnées pendant la phase de désinfection pendant une durée suffisante pour l'élimination des germes présents dans l'espace à désinfecter (3), et
iii. ensuite, expulsion de l'humidité relative hors de l'espace à désinfecter (3) et refroidissement pendant la phase de refroidissement,
la vapeur d'eau produite pour la désinfection étant chassée de l'espace à désinfecter (3) par l'injection d'air filtré pendant la phase de refroidissement.

2. Procédé selon la revendication 1,
dans lequel l'étape ii) est exécutée pendant une durée de plusieurs heures.

3. Procédé selon l'une des revendications précédentes,
dans lequel l'air filtré utilisé pour chasser la vapeur d'eau pendant la phase de refroidissement est produit à partir de l'air ambiant à l'aide de filtres stériles.

4. Procédé selon l'une des revendications précédentes,
dans lequel, pendant au moins une des étapes i), ii) et iii), au moins une des valeurs de mesure suivantes est déterminée et comparée à une valeur de consigne correspondante enregistrée dans une unité de mémoire:
- température dans l'espace à désinfecter (3),
- humidité de l'air dans l'espace à désinfecter (3),
- pression dans l'espace à désinfecter (3).

5. Procédé selon la revendication 4,
dans lequel, lorsqu'un écart de la valeur de mesure par rapport à la valeur de consigne est constaté, une régulation est effectuée pour rapprocher la valeur de mesure de la valeur de consigne.

6. Procédé selon l'une des revendications précédentes,
dans lequel l'armoire climatique (1) comprend un dispositif de stockage d'objets, en particulier un dispositif de stockage d'objets en forme de carrousel, et au moins un dispositif de transport commandés et pilotés par des robots et des capteurs.

7. Procédé selon l'une des revendications précédentes,
dans lequel la pression à l'intérieur de l'espace à désinfecter (3) est équilibrée avec la pression de l'atmosphère ambiante au moyen d'un moyen d'équilibrage de la pression.

8. Utilisation du procédé selon l'une des revendications précédentes pour la désinfection d'armoires climatiques comprenant des internes et des capteurs électroniques.
